# EUROPEAN PATENT SPECIFICATION

(11) **EP 2 925 916 B1**
(45) Date of publication and mention of the grant of the patent: **07.08.2019**
(21) Application number: 13826919.6
(22) Date of filing: 26.11.2013
(51) Int. Cl.: D01D 5/06, C08B 37/08, D01F 9/00, A61L 27/20

(54) **FIBRES BASED ON HYDROPHOBIZED DERIVATIVES OF HYALURONAN, METHOD OF THEIR PREPARATION AND USE, TEXTILES ON BASE THEREOF AND USE THEREOF**
FASERN AUF BASIS VON HYDROPHOBIERTEN HYALURONANDERIVATEN, VERFAHREN ZU DEREN HERSTELLUNG UND VERWENDUNG, TEXTILIEN AUF BASIS DAVON UND VERWENDUNG DAVON
FIBRES BASÉES SUR DES DÉRIVÉS RENDUS HYDROPHOBES D'HYALURONANE, PROCÉDÉ PERMETTANT LEUR PRÉPARATION ET LEUR UTILISATION, TEXTILES À BASE DE CES FIBRES UTILISATION DE CES FIBRES

(30) Priority: 27.11.2012 CZ 20120841
(43) Date of publication of application: 07.10.2015
(73) Proprietor: Contipro a.s., 56102 Dolní Dobrouc (CZ)
(72) Inventor: SCUDLOVA, Jolana, 798 46 Brodek u Konice (CZ); BETAK, Jiri, 569 61 Dolni Ujezd (CZ); WOLFOVA, Lucie, 747 05 Opava (CZ); BUFFA, Radovan, 06601 Humenne (SK); SLEZINGROVA, Klara, 561 63 Nekor (CZ); KLEIN, Pavel, 561 02 Dolní Dobrouc (CZ); MATEJKOVA, Ilona, 562 01 Usti nad Orlici (CZ); BOBEK, Martin, 130 00 Praha 3 (CZ); PITUCHA, Tomas, 544 72 Bila Tremesna (CZ); VELEBNY, Vladimir, 564 01 Zamberk (CZ); SULAKOVA, Romana, 562 01 Usti nad Orlici (CZ)
(74) Representative: Dvorakova, Martina
(86) International application number: PCT/CZ2013/000158
(87) International publication number: WO 2014/082611

(56) References cited:
- JP-A- H0 625 306

## Description

### Field of the invention

The invention relates to the preparation of biodegradable fibres based on hydrophobic derivatives of hyaluronan. The preparation of fibres is performed with the use of the wet spinning method, with the production of continuous monofiles which can be thereafter processed to threads and further to textiles, knitted textiles, or nonwoven textiles. The resulting fibres have excellent processing properties (strength, elongation). Applicable use of these fibres is targeted to medicine, especially to the field of surgical materials, tissue engineering, and controlled release drug systems.

### Background of the invention

Hyaluronan is a linear polysaccharide composed of repeating disaccharide units made of glucuronic acid, which is linked with N-acetyl-glucosamine via β-1,3 glykosidic bond. Disaccharide subunits are joined one to another via β-1,4 bond. It concerns to a substance naturally occurring in the organism where, in particular, it plays a role of organizing the extracellular matrix (EMS). Furthermore, thanks to its interactions with cell receptors, hyaluronan is able not only to bond them to the EMS but also to control them in a particular way via this cell interaction. Furthermore, thanks to its composition, hyaluronan is able to bond substantial amount of water and consequently to hydrate the tissues and therefore ensure not only the transport of nutrients and oxygen to the particular cells but also the transfer of regulation factors between the cells [1]. The fact, that hyaluronan can be synthetized in virtually non-limited amount of a very high purity with the use of streptococcus fermentation, is also very advantageous [2].

Hyaluronan is a substance intrinsic to the body, furthermore it is not immunogenic, teratogenic or otherwise toxic, it interacts with specific cell receptors, strongly hydrates the tissues and lubricates the joints or the eyelid movement on the eye ball.

By these facts, hyaluronan is predetermined for many applications in medicine [3 - 5]. Some of the forms of application, that could be interesting, are various textile products, textiles, knitted textiles or nonwoven textiles for external or internal usage.

However, for these purposes there is a need of fibres that will be able to bond on their surface or carry in their structure biologically active substances, and that will have physical and chemical properties enabling their textile processing. Nowadays, the description of the use of wet spinning for producing the fibres and films from potassium hyaluronate (Rupprecht [6]) or calcium hyaluronate (Sheehan [7]) can be found in the literature. In this case, the spinning is performed into an ethanol solution containing 0.1M KCl or respective calcium salts. The prepared fibres are well soluble in water and even in humid environment they disintegrate, whereas they lose both their shape and their mechanical properties within tens of seconds. The above mentioned properties essentially limit the possibilities of the applicability of native hyaluronan. So far, no preparation using the native fibres prepared from hyaluronan has been on the market.

The preparation of fibres from native hylauronan is protected by patent no. WO 2009/050389**.** The fibres are obtained by precipitation in the bath of concentrated acetic acid (80% to 90%). Then the fibres are dried and elongated. With regard to their preparation from native hyaluronan, the fibres are well soluble in water. The authors state that the solubility can be limited by treatment of the fibres surface with the use of hydrophobic substances of vegetable or animal origin. On the contrary, the fibres which we prepared from hydrophobized hyaluronan are insoluble immediately after their preparation, so there is no need to add other agents later to treat the surface, and therefore the process is considerably simpler from both the technological and economic points of view.

In Patent Application **PV 2010-1001,** an acid of the concentration of 1 to 99 % by weight is also used to prepare the fibres from hyaluronic acid but the acid is mixed with 1 to 99% alcohol to confer higher strength to the fibres. The use of phosphoric acid and metallization baths is also claimed. Formic, acetic, propionic acid mixed with methanol, ethanol, 1-propanol, or 2-propanol is preferred for the coagulation. These fibres are also soluble in comparison with the hydrophobized HA which we used. The same delineation is in Patent Application US2011/0263724 A1.

The above described solubility of native hyaluronan can be removed by using the appropriate chemically modified derivatives based on hyaluronan.

In Patent EP 216453 A2**,** the authors spun hyaluronic acid esters in ethanol+DMSO bath with the use of wet spinning. More specifically, it concerned to ethylester (HYAFF7) and benzylester (HYAFF11). A carboxyl group is esterified, the esterification is proceed by bonding an activated alkyl to carboxyl group of glukuronic acid which is, according to current knowledge [8], important because of the specific bond of hyaluronic acid and cell receptors (CD44).

On the contrary, the derivatives which we result from in the scope of this patent application are modified on hydroxyl groups only, thus the biological properties of a carboxyl group maintain completely preserved as well as in the case of native hyaluronan.

As it is not possible to reach the mechanical properties demanded for the production of a continuous monofile with the use of the bath used in the abovementioned patent, authors of patents use it for producing nonwoven textiles only.

Also the other patents of Fidia Advanced Biopolymers company deal with the production of fibres and nonwoven textiles from hyaluronic acid esters; these patents are as follows: WO 93/11803**,** WO 98/08876**,** US 5658582**,** US 006632802 B2**,** US 2004/0192643 A1**.** The preparation of fibres is the same in all the patents. First the hyaluronic acid esters are dissolved in DMSO, followed by the coagulation into absolute ethanol. The fibres are of the length ranging from 5 to 100 mm. Their shortness is caused by considerable fragility of the fibres; the fragility is caused by the coagulation bath, absolute ethanol, used, as we prove also in our Example 20.

Therefore the authors claim nonwoven textiles only as they are not able to produce a continuous fibre by their technological process, especially with the coagulation bath chosen. In the case of our patent application, the conclusion is the preparation of continuous fibres and their processing into the form of a textile with regular structure (textile, knitted textile) where homogenous distribution of monofilament and higher tensile and flexible properties can be expected.

The mixed fibres, where the hyaluronan derivatives represent a marginal component with the maximum content up to 10 % by weight, are also protected by a patent. The rest of spinning material is made of other polysaccharides, for example gelan, patent application WO 2007/006403**.** The authors are not able to spun hyaluronan as a supporting polymer with the use of their technological process; therefore they add it to the fibres in a small amount to another polymer which is more supporting.

In WO 2007/009728 A2**,** a similar situation is described; the authors spin carboxymethylcellulose with hyaluronic acid derivatives. The components are dissolved in DMSO and spun in a coagulation bath of zinc chloride in ethanol or zinc bromide in ethanol. The authors do not state the amount of zinc remaining in fibres.

However, a higher content of zinc, which is a heavy metal, is cytotoxic. This is proved by the fibres prepared in the Example 6 and by the diagram of viability no. 5 enclosed.

Patent application US 2011/0237539 A1 claims the production of haemostatic fibres based on polysaccharides. The authors report mixtures of the materials: alginate, chitosan, hyaluronic acid, and gelatine. They claim the suspension of sodium hydroxide, methanol, and water as a coagulation solution for the mixture of hyaluronic acid and chitosan. They claim the same bath of sodium hydroxide, methanol, and water for the mixture of gelatine and chitosan. They claim a suspension of calcium chloride, ethanol, and water for spinning the mixture of hyaluronic acid and alginate. However, they also use hyaluronic acid as one of the components, so the hyaluronic acid is not considered to be a supporting polymer of the produced fibres.

Concerning the applications of fibres prepared from hyaluronan esters, the short fibres are patented, especially nonwoven textiles (US 5658582**)**, the short fibres are also used for the preparation of gauzes, materials for sewing, nets, nonwoven textiles (WO 98/08876**,** US 006632802 B2), or also for preparation of bandaging material (WO 2007/003905**)**. Patent application US 2004/0192643 A1 also reports the results from preclinical and clinical study of HYAFF7 and HYAFF 11 materials against postoperative adhesions. The material is tested in the form of a gauze and a nonwoven textile.

The document JP H06 25306 A mentions the possibility of producing a C18-acylated hyaluronic acid derivative and fibres therefrom. However, the Applicant has not succeeded in producing the derivative by following the procedure of this document. References:
1. Radice a kol., Injectablehyaluronic acid derivativewithpharmaceuticals/cells, patent US20020076810 A1.
2. Lee S.A, VanSteenberg M., Rupprecht A.; Spectroscopic studies of the physical properties of hyaluronate films: the origin of the phase transition; Carbohydrate Polymers; 1995; 28; 61-67.
3. Guillaumie F., Furrer P., Felt-Bayens O., Fuhlendorff B. L., Nymand S.; Comparative studies of various hyaluronic acids produced by microbial fermentation for potential topical opthalmic applications. Journal of Biomedical Materials Research Part A; 2009; 1421-1430.
4. Miller R.J., Avila L.Z.; Chemistry and Biology of Hyaluronan : Medicinal Uses of Modified Hyaluronate. Elsevier Ltd. 2004. 505-528.
5. Juhlin L.; Hyaluronan in skin; Journal of Internal Medicine; 1997; 242; 61-66.
6. Rupprecht A.; Wet Spinning of Hyaluronic Acid. Preparation of Oriented Samples; Acta Chemica Scandinavica; 1979; 33; 779-780.
7. Sheehan J.K., Atkins E.D.T., Nieduszynski I.A.; X-ray diffraction studies on the connective tissue polysaccharides; J. Mol. Biol. 1975; 91; 153-163.
8. Banerji S., Wright A.J. a kol.; Structures of the Cd44-hyaluronan comlex provide insight into a fundamental carboxyhydrate-protein interaction; Nature structural and molecular biology; 2007; 14; 234-239.
9. Kas Jan, Kodíček Milan, Valentová Olga; Laboratorní techniky biochemie, VSCHT Praha, 138-139. ISBN: 80-7080-586-2.

### Summary of the Invention

The deficiencies resulting from the state of art, such as immediate solubility of the fibres prepared from native hyaluronan, are solved by this invention by the use of hydrophobized hyaluronan. Hyaluronan is preferably modified on primary alcohol of N-acetyl-glucosamine but the acylation can occur also on secondary alcohols of glucuronic acid. Anhydrides of fatty acids with the chain of a length preferably of C₁₁ - C₁₈ serve as the acylation agents. The degree of substitution of the derivatives useful for the spinning is:
for C₁₁ - C₁₂ derivatives preferably 30 % and more,
for C₁₃ - C₁₅ derivatives preferably 20 % and more,
for C₁₆ - C₁₈ derivatives preferably 5 % and more.

According to the invention, the method of the preparation of the fibres on the base of hyaluronan C₁₁ - C₁₈ -acylated on hydroxyl groups of hyaluronic acid, preferably acylated on a primary alcohol of N-acetyl-glucosamine, is realized by the preparation of a spinning solution comprising acylated hyaluronan derivative of the concentration ranging from 0.01 % by weight to 15 % by weight in a mixture of water and 2-propanol, where the water content ranges from 30 % by volume to 90 % by volume, 2-propanol content is 10 % by volume to 70 % by volume. In a preferred embodiment a derivative based on palmitoyl-hyaluronan is used. This spinning solution is introduced to the coagulation bath containing the water solution of organic acid or its salts of alkali metals, or alkaline earth metals, where the acid is selected from the group comprising lactic, tartaric, citric, malic, ascorbic, oxalic acid or a combination thereof, to form fibres. Then the fibres are washed with alcohol, for example ethanol, 1-propanol or 2-propanol, and dried. Eventually the fibres can be elongated after the washing. The concentration of the water solution of organic acid in the coagulation bath is 0.5 % to 30 % by weight for lactic acid, 0.01 % to 57 % by weight for tartaric acid, 0.5 % to 42 % by weight for citric acid, 0.5 % to 50 % by weight for malic acid, 0.5 % to 25 % by weight for ascorbic acid, and 0.5 % to 60 % by weight for oxalic acid. Preferably the coagulation bath contains a saturated water solution of organic acid. In one preferred embodiment the coagulation bath contains the saturated water solution of lactic acid. Preferably the spinning solution is deaerated before its introduction into the coagulation bath. The temperature of the coagulation bath ranges from 15 to 45 °C and the time of keeping the fibres in the coagulation bath ranges from 10 seconds to 24 hours.

Further the invention relates to the fibres based on the derivative of hyaluronan C₁₁ - C₁₈-acylated on hydroxyl groups of hyaluronic acid, the fibres have dimensions of 50 to 300 µm, and the tensile strength in the range of 0.3 to 1 cN/dtex, and the tensibility in the range of 10 to 40 %. The fibres can be used for the production of knitted, woven, or nonwoven textiles. Textiles made from the fibres of the invention can be used for example for the production of implantable surgical materials for human and veterinary medicine.

What is important is that in comparison with the patents EP 216453 A2, WO 93/11803, WO 98/08876, US 5658582, US 006632802 B2, and US 2004/0192643 A1 all the present carboxyl groups of hyaluronic acid, which are responsible for biological properties, are preserved.

Furthermore, newer and more effective precipitation baths that are less harmful for human's health and environment are used. It concerns non-toxic, non-explosive, and non-flammable solutions, in comparison with the baths used in PV 2010-1001.

| | **Hazard class** | **H-phrases** | **P-phrases** |
|---|---|---|---|
| Formic acid according to PV2010-1001 | C, flammable | H226, H314 | P210, P260, P280, P305+P351+P338, P301+P330+P331 |
| Acetic acid according to PV2010-1001 | C, flammable | H226, H314 | P210, P260, P280, P305+P351+P338, P301+P330+P332 |
| Propionic acid according to PV2010-1001 | C | H314 | P280, P301+P330+P331, P305+P351+P338 |

| | **Hazard class** | **H-phrases** | **P-phrases** |
|---|---|---|---|
| Citric acid | Xi | H319 | P280, P305+P351+P338 |
| D,L-malic acid | Xn, Xi | H302, H318, H315, H335 | P261, P280, P305+P351+P338 |
| Tartaric acid | Xi | H319, H335, H315 | P280, P305+P351+P338 |
| Lactic acid | Xi | H315, H318 | P280, P305+P351+P338 |
| Ascorbic acid | - | - | - |
| Oxalic acid | Xn | H302, H312 | P302+P352 |

The fibres from hydrophobized hyaluronan have considerably better properties for textile processing in comparison with the fibres form native hyaluronan; this is caused mainly by enhanced tensibility. In this embodiment, the fibres do not beak anymore and it is possible to produce the fibres of the length of hundreds meters or kilometres, whereas the fibres can be further processed on operation machines by common textile procedures as is weaving, knitting, etc.

The tensibility is very important for fibre processing. During weaving, a dynamical stress occurs on a warp and a weft, during forming the shed and weft propulsion.

During knitting, the dynamical stress of the thread occurs at the retraction of bobbin and at tightening the loop [Study texts, Technical University of Liberec]. The values of fibres tensibility are given mainly by the derivative used, and the disposition for good tensile results from its chemical structure [Rogovin Z.A. Chemie a technologie umělých vláken, 1956].

The use of claimed organic acids as precipitation baths is not clearly obvious as in comparison with the abovementioned acids in the patent application PV2010-1001 (formic, acetic, propionic acid) these acids are solid. The attempts to prepare the fibres in water or alcohol only were not successful. Therefore it was necessary to find empirically the mixtures that are the most useful for the strength and the tensibility of fibres so that it would be possible to process the obtained fibres on operational textile machines.

Another example of ambiguity is the use of lactic acid for a precipitation bath. Lactic acid is commonly available as 80% water solution, this solution being very viscous; therefore it is not possible to precipitate the fibres into this solution.

### Experimentally, this concentration composition has been found optimal:

lactic acid - preferably 0.5% - 30% water solution
tartaric acid - preferably 0.01% to saturated solution (57%)
citric acid - preferably 0.5% to saturated solution (42%)
malic acid - preferably 0.5% - 50%
ascorbic acid - preferably 0.5% to saturated solution (25%)
or any combination thereof.

Surprisingly, when using a stronger acid (formic, citric, lactic, malic, tartaric) it could be expected that the strength of acid would influence the reduction of molecular weight of hyaluronic acid derivative (acid hydrolysis of saccharides). The reduction of molecular weight leads to the reduction of fibre strength [Hladik V., Textilní vlákna, 1970]. However, the fibres from hydrophobized HA being produced were not successfully wind up with the use of winding rollers for further operations, when the water solution of formic acid or the mixture of formic acid and alcohol were used. The fibre broke during the attempt to wind it up (increase of needs of strength). Low strength is caused by decrease of molecular weight.

However, with the use of the claimed baths (tartaric, citric, lactic, malic, ascorbic acid) the strength is not low. The results of measurement, with the use of SEC-MALLS, of the fibres prepared with the use of various baths show that Mw of the fibres prepared with the use of citric, lactic, malic, tartaric acid decreases but the properties of the fibres are preserved.

| | **prepared according to** | **Mw HA [kDa]** | **p Ka** | **Strength [cN/tex]** | **tensibility [%]** |
|---|---|---|---|---|---|
| hydrophobized derivative of HA | Example 1 | 253 | - | - | - |
| formic acid | Example 24 | 88 | 3.75 | not possible to measure | |
| acetic acid | Example 26 | 167 | 4.76 | 5.60 | 16.13 |
| propionic acid | Example 25 | 193 | 4.88 | 3.00 | 11.00 |
| ascorbic acid | Example 10 | 120 | 4.1 | 9.37 | 21.07 |
| lactic acid | Example 12 | 137 | 3.86 | 5.77 | 21.90 |
| malic acid | Example 11 | 130 | 3.46 | 7.50 | 25.83 |
| citric acid | Example 5 | 106 | 3.15 | 7.80 | 20.53 |
| tartaric acid | Example 8 | 95 | 3.01 | 4.93 | 16.49 |

Therefore the summary of the invention lies in the use of the mentioned hydrophobized derivatives of hyaluronan in the combination with the mentioned baths and due to it these fibres show new enhanced properties in the form of insoluble, biodegradable, continuous fibres with enhanced tensibility. Due to this these fibres can be processed by conventional textile techniques such as weaving, knitting, etc.

The processing can be carried out on conventional operational apparatuses (Example 29, which has not been possible so far for the fibres from native HA or HA derivatives. In the patent application PV 2010-1001 only handmade textiles are reported. However, there are higher demands for strength and especially tensibility of the fibres processed by textile machines.

As for the degradability in an organism, palmitoyl hyaluronan seems to be the most important starting hyaluronan derivative. Palmitic acid is a part of animal (body) fat. Palmitane chain normally degrades by β-oxidation in a body.

The fibres are prepared with the use of wet spinning method. The coagulation baths for preparation of these fibres are specific and they are composed of:
- water solutions of organic acids,
- water solutions of organic acids salts,
- or any combination thereof.

The concentration of solutions for fibre preparation ranges from 0.01 % to saturated solutions.

The fibres produced from a precipitation bath are further washed with lower alcohol (ethanol, 1-propanol, 2-propanol) and preferably they can be drawn. The produced fibres have the diameter of 50 - 300 µm, according to spinning parameters used (spinning nozzle, extrusion speed and speed of winding, drawing ratio) and they are produced as continuous filaments and further twisted into the form of twisted cables suitable for machine processing by textile techniques. The fibres are water insoluble, they only swell. They do not show any cytotoxic properties and they are enzymatically degradable. Both the strength and tensile are suitable for further processing of the fibres, such as twisting into threads, or for preparing the knitted, woven or nonwoven textiles.

### Brief description of the drawings

Fig. 1 shows a monofile prepared according to the Expamle 12, the monofile is imaged with the use of scanning electron microscopy.

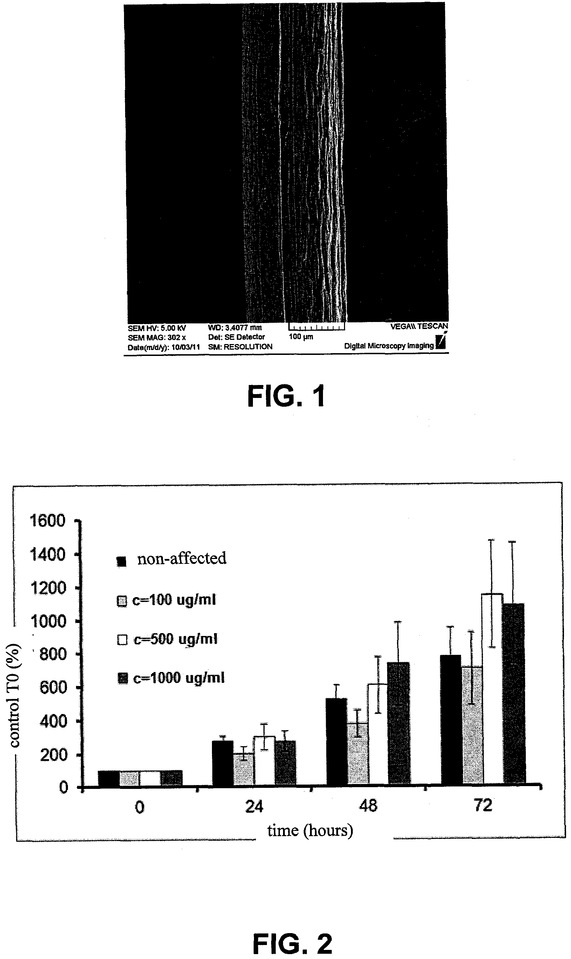

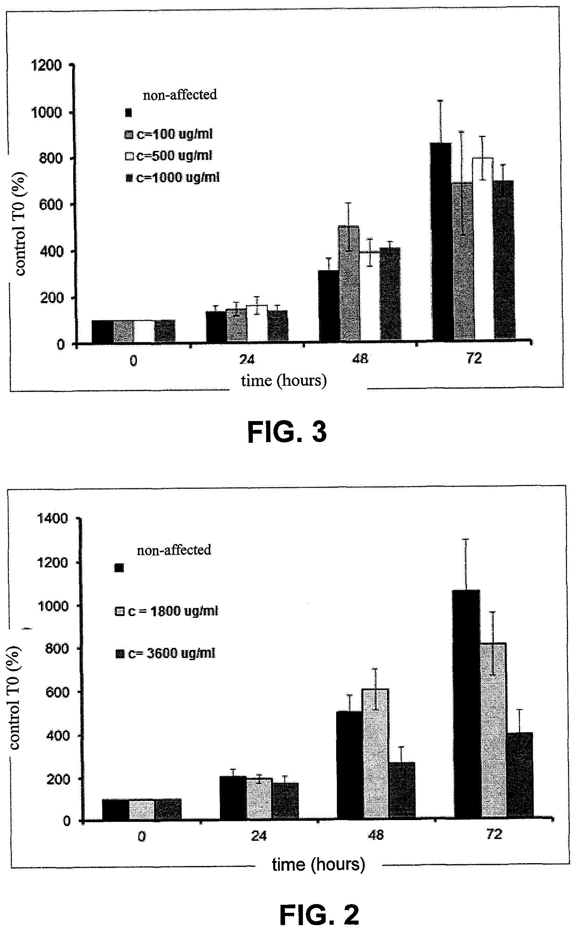

Fig. 2 and Fig. 3 show the diagrams presenting the influence of derivatives used for the production of fibres on cell viability. Specifically, the Fig. 2 shows the results of the tests of influence of palmitoyl hyaluronan derivative prepared according to the Example 1 on viability. In the diagram, the cell viability (percent of control in time 0) versus time (hours) is plotted. Fig. 3 shows the results of the tests of the influence of palmitoyl hyaluronan derivative prepared according to the Example 4 on viability. In the diagram, the cell viability (percent of control in time 0) versus time (hours) is plotted.

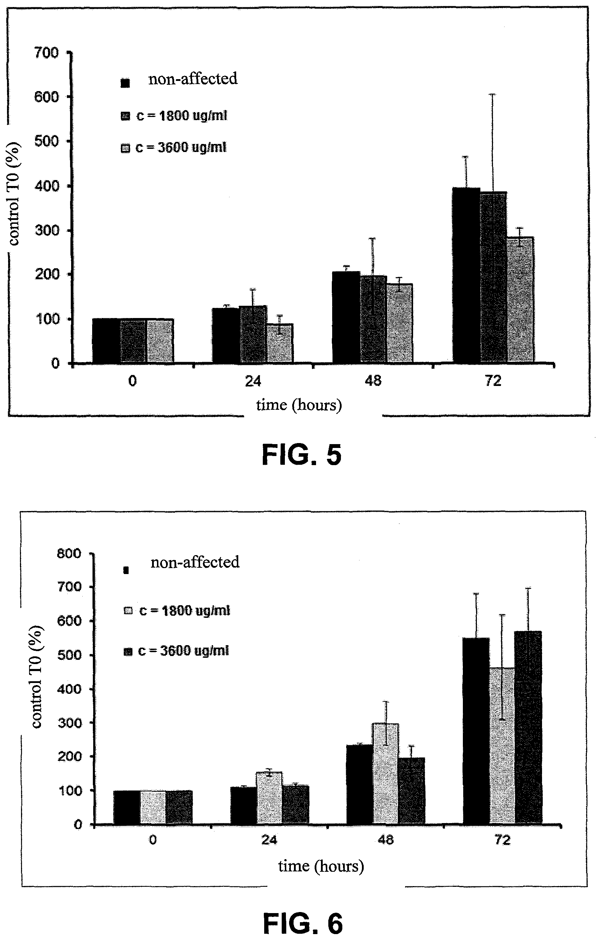

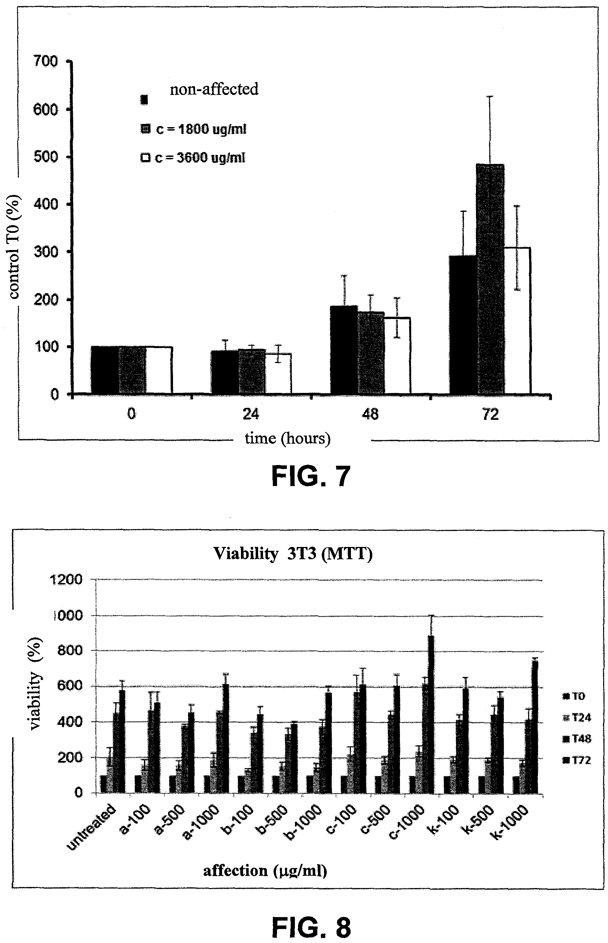

Fig. 4 to 8 show the diagrams presenting the results of the influence of the fibres produced from various precipitation baths on the cell viability. Specifically, Fig. 4 shows the results of the influence of the fibres prepared according to the Example 3 (the precipitation bath is a water solution of citric acid) on the cell viability, Fig. 5 shows the results of the influence of the fibres prepared according to the Example 12 (the precipitation bath is a water solution of lactic acid) on the cell viability, Fig. 6 shows the results of the influence of the fibres prepared according to the Example 8 (the precipitation bath is a water solution of tartaric acid) on the cell viability, Fig. 7 shows the results of the influence of the fibres prepared according to the Example 11 (the precipitation bath is a water solution of malic acid) on the cell viability.
Fig. 8 shows the diagram presenting the influence of fibre degradation products on the cell viability.
Fig. 9 shows the threads from palmitoyl hyaluronan. The thread on the left is from two monofiles, the thread on the right is from three monofiles.
Fig. 10 shows the illustration of processing the fibres from hydrophobic hyaluronan into an one-face weft-knitted fabric (the white part).
Fig. 11 shows the illustration of processing the fibres from hydrophobic hyaluronan into a double weft-knitted fabric.
Fig. 12 shows the illustration of processing the fibres from hydrophobic hyaluronan into a tubular fabric.

### Preferred embodiments of the invention

The molecular weight was measured with the use of HPLC from Shimadzu with the miniDAWN light scattering detector from Watt Technologies (so-called SEC-MALLS method). The said molecular weights are weight average, unless otherwise stated.

The identification and the determination of the degree of the substitution were made with the use of NMR. The degree of the substitution (DS) was calculated from the integral of signals of an appropriate substituent (i.e. C16 acyl) and the signal of CH₃-GlcN at 2.0 ppm. The degree of the substitution of 100 % corresponds to bonding one substituent onto one hyaluronan dimer unit. The NMR spectra of the derivatives were measured on BRUKER AVANCE 500 (500 MHz) in D₂O, in the mixture of D₂O/2-propanol-d₈ in the rate of 4:1. Chemical shifts were calibrated on the inner standard of deuterized 3-trimethylsilylpropanoic acid (TSPA). Following spectra were measured: ¹H NMR, ¹³C NMR, HH COSY, HSQC and DOSY-NMR. The software TOPSPIN 1.2 from Bruker, and software SpinWorks 3.1 were used for experimental data processing.

The acylated derivatives (palmitoyl hyaluronan, stearoyl hyaluronan) were prepared in a similar manner according to the Example 1. The degree of the substitution depends on the equivalents used, the said range is as follows:

| HDA 0,5 eq | HDA 1 eq | HDA 2 eq |
|---|---|---|
| DS ≤12 % | DS 12 % - 30 % | DS 35 % - 55 % |

The acylated derivatives of undecanoyl hyaluronan were prepared in a similar manner according to the Example 2.

The images from the scanning electron microscope were made with the use of Tescan VEGA II LSU machine with wolfram cathode and at maximum resolution of 3 nm. The accelerating voltage of the primary beam was 5 kV, work distance 3 = 4 mm, the images were made in the high vacuum regime.

### Example 1

Sodium hyaluronan (HA-Na, 15 g; 250 kDa) was dissolved in 300 ml of distilled water, after its complete dissolving 300 ml of tetrahydrofuran (THF) was added to the solution. Then triethylamine (TEA) (2.5 eq), dimethylaminopyridine (DMAP) (0.04 eq), and palmitoic acid anhydride (HDA) (2 eq) were added to the solution. This solution was stirred for 2 h at room temperature and then 0.5 h at 45 °C (temperature of the solution). Then 300 ml of distilled water with 5 eq of NaCl (on HA-Na basis) and 300 ml of distilled water for washing the reaction flask was added into the homogenous solution. The solution was stirred and then precipitated with 900 ml of 100% 2-propanol, washed once with 100% 2-propanol (500 ml), and 4 times with 80% 2-propanol (500 ml), and 2 times with 100% 2-propanol (500 ml). A wet derivative was dried in a drying oven for 48 hours at 40 °C. The derivative of palmitoyl hyaluronan prepared thereby showed the degree of the substitution of 36 % (determined with NMR), and Mw of 250 kDa.

### Example 2

5.0 g of sodium hyaluronan (12.5 mmol, 250 kDa) was dissolved in 100 ml of distilled water. Then 70 ml of THF was added. Then TEA (7.0 ml; 4 eq.) and DMAP (153.0 mg; 0.1 eq.) were added into the solution. At the same undecanoic acid was dissolved (9.3 g; 4 eq) in 30 ml of THF, then TEA (7.0 ml; 4 eq.) and ethyl-chlorformiate (4.76 ml, 4 eq.) were added to the solution. The acid was activated at 0 - 5 °C for 15 min. After the activation of the acid, the precipitate, i.e. the mixed anhydride of udecanoic acid and ethyl-chlorformiate, was filtered into the prepared solution of HA. The reaction was run for 5 hours at room temperature. Then the reaction mixture was diluted with 50 ml of water with 4.0 g of NaCl. The acylated derivative was isolated from the reaction mixture by precipitation with the use of 4-fold of absolute 2-propanol. After decantation, the precipitate was repeatedly washed, first with water solution of 2-propanol (80% by volume) and then with absolute 2-propanol. Then the precipitate was dried for 48 hours at the temperature of 40 °C. The degree of the substitution was determ ined to be 37 % (determined by NMR).

### Example 3

The charge of 1 g of palmitoyl hyaluronan with the degree of the substitution of 36 % and Mw of 250 kDa was dissolved in the mixture of 10 ml of demi-water and 10 ml of 2-propanol. The resulting 5.3% solution was deaerated by centrifugation and introduced to the coagulation bath with the use of NEXUS 6000 charger at the extrusion speed of 200 µl·min⁻¹. The coagulation bath contained saturated water solution of citric acid of the room temperature. The fibre passed through the bath of the length of 0.4 m at the speed of 1.2 m·min⁻¹ and was coned and wound, with the use of a set of winding rollers, on a roller partially immersed into 100% 2-propanol, where it was washed, for approximately 1 hour.

### Example 4

The charge of 1.3 g of palmitoyl hyaluronan with the degree of the substitution of 15 % and Mw of 250 kDa was dissolved in the mixture of 10 ml of demi-water and 10 ml of 2-propanol. The resulting 6.8% solution was deaerated by centrifugation and introduced to the coagulation bath with the use of NEXUS 6000 charger at the extrusion speed of 200 µl·min⁻¹. The coagulation bath contained saturated water solution of citric acid of the room temperature. The fibre passed through the bath of the length of 0.4 m at the speed of 1.2 m·min⁻¹ and was coned and wound, with the use of a set of winding rollers, on a roller partially immersed into 100% 2-propanol, where it was washed, for approximately 1 hour.

### Example 5

The charge of 1.32 g of palmitoyl hyaluronan with the degree of the substitution of 46 % and Mw of 250 kDa was dissolved in the mixture of 11 ml of demi-water and 11 ml of 2-propanol. The resulting 6.3% solution was deaerated by centrifugation and introduced to the coagulation bath with the use of NEXUS 6000 charger at the extrusion speed of 200 µl·min⁻¹. The coagulation bath contained saturated water solution of citric acid of the room temperature. The fibre passed through the bath of the length of 0.4 m at the speed of 1.2 m·min⁻¹ and was coned and wound, with the use of a set of winding rollers, on a roller partially immersed into 100% 2-propanol, where it was washed, for approximately 1 hour.

### Example 6

The charge of 0.95 g of stearoyl hyaluronan with the degree of the substitution of 35 % and Mw of 130 kDa was dissolved in the mixture of 10 ml of demi-water and 10 ml of 2-propanol. The resulting 5% solution was deaerated by centrifugation and introduced to the coagulation bath with the use of NEXUS 6000 charger at the extrusion speed of 200 µl·min⁻¹. The coagulation bath contained saturated water solution of citric acid of the room temperature. The fibre passed through the bath of the length of 0.4 m at the speed of 1.2 m·min⁻¹ and was coned and wound, with the use of a set of winding rollers, on a roller partially immersed into 100% 2-propanol, where it was washed, for approximately 1 hour.

### Example 7

The charge of 0.5 g of undecanoyl hyaluronan with the degree of the substitution of 37 % and Mw of 250 kDa was dissolved in the mixture of 10 ml of demi-water and 10 ml of 2-propanol. The resulting 2.7% solution was deaerated by centrifugation and introduced to the coagulation bath with .the use of NEXUS 6000 charger at the extrusion speed of 200 µl·min⁻¹. The coagulation bath contained saturated water solution of citric acid with the room temperature. The fibre passed through the bath of the length of 0.4 m at the speed of 1.2 m·min⁻¹ and was coned and wound, with the use of a set of winding rollers, on a roller partially immersed into 100% 2-propanol, where it was washed, for approximately 1 hour.

### Example 8

The charge of 1.32 g of palmitoyl hyaluronan with the degree of the substitution of 46 % and Mw of 250 kDa was dissolved in the mixture of 11 ml of demi-water and 11 ml of 2-propanol. The resulting 6.3% solution was deaerated by centrifugation and introduced to the coagulation bath with the use of NEXUS 6000 charger at the extrusion speed of 200 µl·min⁻¹. The coagulation bath contained saturated water solution of tartaric acid of the room temperature. The fibre passed through the bath of the length of 0.4 m at the speed of 1.2 m·min⁻¹ and was coned and wound, with the use of a set of winding rollers, on a roller partially immersed into 100% 2-propanol, where it was washed, for approximately 1 hour.

### Example 9

The charge of 1.1 g of palmitoyl hyaluronan with the degree of the substitution of 52% and Mw of 250 kDa was dissolved in the mixture of 10 ml of demi-water and 10 ml of 2-propanol. The resulting 5.7% solution was deaerated by centrifugation and introduced to the coagulation bath with the use of NEXUS 6000 charger at the extrusion speed of 200 µl·min⁻¹. The coagulation bath contained saturated water solution of citric acid of the room temperature. The fibre passed through the bath of the length of 0.4 m at the speed of 1.2 m·min⁻¹ and was coned and wound, with the use of a set of winding rollers, on a roller partially immersed into 100% 2-propanol, where it was washed, for approximately 1 hour.

### Example 10

The charge of 1.32 g of palmitoyl hyaluronan with the degree of the substitution of 46 % and Mw of 250 kDa was dissolved in the mixture of 11 ml of demi-water and 11 ml of 2-propanol. The resulting 6.3% solution was deaerated by centrifugation and introduced to the coagulation bath with the use of NEXUS 6000 charger at the extrusion speed of 200 µl·min⁻¹. The coagulation bath contained saturated water solution of ascorbic acid of the room temperature. The fibre passed through the bath of the length of 0.4 m at the speed of 1.2 m·min⁻¹ and was coned and wound, with the use of a set of winding rollers, on a roller partially immersed into 100% 2-propanol, where it was washed, for approximately 1 hour.

### Example 11

The charge of 1.32 g of palmitoyl hyaluronan with the degree of the substitution of 46 % and Mw of 250 kDa was dissolved in the mixture of 11 ml of demi-water and 11 ml of 2-propanol. The resulting 6.3% solution was deaerated by centrifugation and introduced to the coagulation bath with the use of NEXUS 6000 charger at the extrusion speed of 200 µl·min⁻¹. The coagulation bath contained saturated water solution of malic acid of the room temperature. The fibre passed through the bath of the length of 0.4 m at the speed of 1.2 m·min⁻¹ and was coned and wound, with the use of a set of winding rollers, on a roller partially immersed into 100% 2-propanol, where it was washed, for approximately 1 hour.

### Example 12

The charge of 1.32 g of palmitoyl hyaluronan with the degree of the substitution of 46 % and Mw of 250 kDa was dissolved in the mixture of 11 ml of demi-water and 11 ml of 2-propanol. The resulting 6.3% solution was deaerated by centrifugation and introduced to the coagulation bath with the use of NEXUS 6000 charger at the extrusion speed of 200 µl·min⁻¹. The coagulation bath contained 20% water solution of lactic acid of the room temperature. The fibre passed through the bath of the length of 0.4 m at the speed of 1.2 m·min⁻¹ and was coned and wound, with the use of a set of winding rollers, on a roller partially immersed into 100% 2-propanol, where it was washed, for approximately 1 hour.

### Example 13

The charge of 1.32 g of palmitoyl hyaluronan with the degree of the substitution of 46 % and Mw of 250 kDa was dissolved in the mixture of 11 ml of demi-water and 11 ml of 2-propanol. The resulting 6.3% solution was deaerated by centrifugation and introduced to the coagulation bath with the use of NEXUS 6000 charger at the extrusion speed of 200 µl·min⁻¹. The coagulation bath contained 20% water solution of lactic acid of the room temperature. The fibre passed through the bath of the length of 0.4 m at the speed of 1.2 m·min⁻¹ and was coned and wound, with the use of a set of winding rollers, on a roller partially immersed into 100% 2-propanol, where it was washed, for approximately 1 hour. The last roller was speeded up to1.4 m.min⁻¹ in comparison with the one next to the last, so the fibres were drawn of about 20 %.

### Example 14

The charge of 1.32 g of palmitoyl hyaluronan with the degree of the substitution of 46 % and Mw of 250 kDa was dissolved in the mixture of 11 ml of demi-water and 11 ml of 2-propanol. The resulting 6.3% solution was deaerated by centrifugation and introduced to the coagulation bath with the use of NEXUS 6000 charger at the extrusion speed of 200 µl·min⁻¹. The coagulation bath contained 50% water solution of oxalic acid of the room temperature. The fibre passed through the bath of the length of 0.4 m at the speed of 1.2 m·min⁻¹ and was coned and wound, with the use of a set of winding rollers, on a roller partially immersed into 100% 2-propanol, where it was washed, for approximately 1 hour.

### Example 15

The charge of 1.3 g of palmitoyl hyaluronan with the degree of the substitution of 15 % and Mw of 250 kDa was dissolved in the mixture of 10 ml of demi-water and 10 ml of 2-propanol. The resulting 6.8% solution was deaerated by centrifugation and introduced to the coagulation bath with the use of NEXUS 6000 charger at the extrusion speed of 200 µl·min⁻¹. The coagulation bath contained the solution of lactic acid composed of: 80 % by weight of 2-propanol, 18 % by weight of lactic acid, 2 % by weight of water of the room temperature. The fibre passed through the bath of the length of 0.4 m at the speed of 1.2 m·min⁻¹ and was coned and wound, with the use of a set of winding rollers, on a roller partially immersed into 100% 2-propanol, where it was washed, for approximately 1 hour.

### Example 16

The charge of 0.95 g of stearoyl hyaluronan with the degree of the substitution of 35 % and Mw of 130 kDa was dissolved in the mixture of 10 ml of demi-water and 10 ml of 2-propanol. The resulting 5% solution was deaerated by centrifugation and introduced to the coagulation bath with the use of NEXUS 6000 charger at the extrusion speed of 200 µl·min⁻¹. The coagulation bath contained the solution of lactic acid composed of: 80 % by weight of 2-propanol, 18 % by weight of lactic acid, 2 % by weight of water of the room temperature. The fibre passed through the bath of the length of 0.4 m at the speed of 1.2 m·min⁻¹ and was coned and wound, with the use of a set of winding rollers, on a roller partially immersed into 100% 2-propanol, where it was washed, for approximately 1 hour.

### Example 17

The charge of 0.5 g of undecanoyl hyaluronan with the degree of the substitution of 37 % and Mw of 250 kDa was dissolved in the mixture of 10 ml of demi-water and 10 ml of 2-propanol. The resulting 2.7% solution was deaerated by centrifugation and introduced to the coagulation bath with the use of NEXUS 6000 charger at the extrusion speed of 200 µl·min⁻¹. The coagulation bath contained the solution of lactic acid composed of: 80 % by weight of 2-propanol, 18 % by weight of lactic acid, 2 % by weight of water of the room temperature. The fibre passed through the bath of the length of 0.4 m at the speed of 1.2 m·min⁻¹ and was coned and wound, with the use of a set of winding rollers, on a roller partially immersed into 100% 2-propanol, where it was washed, for approximately 1 hour.

### Example 18 (comparative Example)

The charge of 1.3 g of palmitoyl hyaluronan with the degree of the substitution of 15 % and Mw of 250 kDa was dissolved in the mixture of 10 ml of demi-water and 10 ml of 2-propanol. The resulting 6.8% solution was deaerated by centrifugation and introduced to the coagulation bath with the use of NEXUS 6000 charger at the extrusion speed of 200 µl·min⁻¹. The coagulation bath contained the solution composed of 33 % by weight of benzoic acid and 67 % by weight of 2-propanol of the room temperature. The fibre passed through the bath of the length of 0.4 m at the speed of 1.2 m·min⁻¹ and was coned and wound, with the use of a set of winding rollers, on a roller partially immersed into 100% 2-propanol, where it was washed, for approximately 1 hour.

### Salts of organic acids

### Example 19

The charge of 1 g of palmitoyl hyaluronan with the degree of the substitution of 36 % and Mw of 250 kDa was dissolved in the mixture of 10 ml of demi-water and 10 ml of 2-propanol. The resulting 5.3% solution was deaerated by centrifugation and introduced to the coagulation bath with the use of NEXUS 6000 charger at the extrusion speed of 200 µl·min⁻¹. The coagulation bath contained the saturated water solution of (tri)sodium citrate of the room temperature. The fibre passed through the bath of the length of 0.4 m at the speed of 1.2 m·min⁻¹ and was coned and wound, with the use of a set of winding rollers, on a roller partially immersed into 100% 2-propanol, where it was washed, for approximately 1 hour.

### Example 20 (comparative Example)

The charge of 1 g of palmitoyl hyaluronan with the degree of the substitution of 36 % and Mw of 250 kDa was dissolved in the mixture of 10 ml of demi-water and 10 ml of 2-propanol. The resulting 5.3% solution was deaerated by centrifugation and introduced to the coagulation bath with the use of NEXUS 6000 charger at the extrusion speed of 200 µl·min⁻¹. The coagulation bath contained the saturated water solution of magnesium acetate of the room temperature. The fibre passed through the bath of the length of 0.4 m at the speed of 1.2 m·min⁻¹ and was coned and wound, with the use of a set of winding rollers, on a roller partially immersed into 100% 2-propanol, where it was washed, for approximately 1 hour.

### Example 21 (comparative Example)

The charge of 1 g of palmitoyl hyaluronan with the degree of the substitution of 36 % and Mw of 250 kDa was dissolved in the mixture of 10 ml of demi-water and 10 ml of 2-propanol. The resulting 5.3% solution was deaerated by centrifugation and introduced to the coagulation bath with the use of NEXUS 6000 charger at the extrusion speed of 200 µl·min⁻¹. The coagulation bath contained the saturated water solution of sodium acetate of the room temperature. The fibre passed through the bath of the length of 0.4 m at the speed of 1.2 m·min^{-*1} and was coned and wound, with the use of a set of winding rollers, on a roller partially immersed into 100% 2-propanol, where it was washed, for approximately 1 hour.

### Comparative Examples 22 - 24

### Example 22

Comparison with the bath used in the documents WO 93/11803, WO 98/08876, US 5658582, US 006632802 B2, US 2004/0192643 A1.

The charge of 1 g of palmitoyl hyaluronan with the degree of the substitution of 36 % and Mw of 250 kDa was dissolved in the mixture of 10 ml of demi-water and 10 ml of 2-propanol. The resulting 5.3% solution was deaerated by centrifugation and introduced to the coagulation bath with the use of NEXUS 6000 charger at the extrusion speed of 200 µl·min⁻¹. The coagulation bath contained absolute ethanol solution. The resulting fibre could not be coned by the set of winding rollers, as the fibre was breaking. The pieces of the length of maximum 100 mm were obtained and taken out with the use of a forceps.

### Example 23

Comparison with the bath used in the documents WO2009/050389, PV 2010-1001.

The charge of 1.32 g of palmitoyl hyaluronan with the degree of the substitution of 46 % and Mw of 250 kDa was dissolved in the mixture of 11 ml of demi-water and 11 ml of 2-propanol. The resulting 6.3% solution was deaerated by centrifugation and introduced to the coagulation bath with the use of NEXUS 6000 charger at the extrusion speed of 200 µl·min⁻¹. The coagulation bath contained alcohol solution composed of 98% acetic acid and 100% 2-propanol (ratio 4:1 by volume) of the room temperature. The fibre passed through the bath of the length of 0.4 m at the speed of 1.2 m·min⁻¹ and was coned and wound, with the use of a set of winding rollers, on a roller partially immersed into 100% 2-propanol, where it was washed, for approximately 1 hour.

### Example 24

Comparison with the bath used in the document PV2010-1001.

The charge of 1.32 g of palmitoyl hyaluronan with the degree of the substitution of 46 % and Mw of 250 kDa was dissolved in the mixture of 11 ml of demi-water and 11 ml of 2-propanol. The resulting 6.3% solution was deaerated by centrifugation and introduced to the coagulation bath with the use of NEXUS 6000 charger at the extrusion speed of 200 µl·min⁻¹. The coagulation bath formed of alcohol solution composed of 98% formic acid and 100% 2-propanol (ratio 4:1 by volume) of the room temperature. The fibre passed through the bath of the length of 0.4 m at the speed of 1.2 m·min⁻¹ and it was coned with the use of a set of winding rollers. The fibre was breaking during the coning very often (lack of the strength); the pieces of the length of 300 mm were obtained.

### Example 25

The charge of 1.32 g of palmitoyl hyaluronan with the degree of the substitution of 46 % and Mw of 250 kDa was dissolved in the mixture of 11 ml of demi-water and 11 ml of 2-propanol. The resulting 6.3% solution was deaerated by centrifugation and introduced to the coagulation bath with the use of NEXUS 6000 charger at the extrusion speed of 200 µl·min⁻¹. The coagulation bath contained the water solution of 80% propionic acid of the room temperature. The fibre passed through the bath of the length of 0.4 m at the speed of 1.2 m·min⁻¹ and was coned and wound, with the use of a set of winding rollers, on a roller partially immersed into 100% 2-propanol, where it was washed, for approximately 1 hour.

### Example 26

The charge of 1.32 g of palmitoyl hyaluronan with the degree of the substitution of 46 % and Mw of 250 kDa was dissolved in the mixture of 11 ml of demi-water and 11 ml of 2-propanol. The resulting 6.3% solution was deaerated by centrifugation and introduced to the coagulation bath with the use of NEXUS 6000 charger at the extrusion speed of 200 µl·min⁻¹. The coagulation bath contained water solution of 80% acetic acid of the room temperature. The fibre passed through the bath of the length of 0.4 m at the speed of 1.2 m·min⁻¹ and was coned and wound, with the use of a set of winding rollers, on a roller partially immersed into 100% 2-propanol, where it was washed, for approximately 1 hour.

### Example 27

The charge of 1.32 g of palmitoyl hyaluronan with the degree of the substitution of 46 % and Mw of 250 kDa was dissolved in the mixture of 11 ml of demi-water and 11 ml of 2-propanol. The resulting 6.3% solution was deaerated by centrifugation and introduced to the coagulation bath with the use of NEXUS 6000 charger at the extrusion speed of 200 µl·min⁻¹. The coagulation bath contained the water solution of 80% formic acid of the room temperature. The fibre passed through the bath of the length of 0.4 m at the speed of 1.2 m·min⁻¹ and it was coned with the use of a set of winding rollers. The fibre was breaking during the coning very often (lack of the strength); the pieces of the length of 300 mm were obtained.

### Example 28

Biodegradability of the fibres was tested in the enzyme medium of hyaluronidase (bovine testicular hyaluronidase, Sigma Aldrich) and esterase (rabbit liver enzyme, Sigma Aldrich).

The method used for studying the fibres degradation at the presence of enzymes is the determination of soluble portion (hylauronane oligosaccharides) with the use of Somogyi-Nelson agent [9]. The determination of the enzymatic degradation of fibres was performed as follows:
The fibres prepared in a similar way as in the Example 12 were metered to the constant length of 400 mm, with the constant weight of 10 mg, coiled into a jacquard leash, placed into the 500µl Eppendorf microtube and embedded by sterile PBS for 24 hour to wash the fibres. After sucking the washing PBS, a fresh buffer (500 µl) was added to the tube. 3 samples were prepared form each fibre:
A) the fibre in PBS only (control);
B) the fibre in PBS with bovine testicular hyaluronidase (200 U);
C) the fibre in PBS with hyaluronidaze (BTH; 200 U) with rabbit liver esterase.

As the enzymes can also act as a source of reductive ends, the background control, i.e. PBS only, PBS+BTH, and PBS+BTH+CHE, was prepared for all the fibres. During the measurement, the absorbance of the background was subtracted from the absorbance of the samples with the fibres.

After adding the enzymes to the fibres and stirring, the samples were taken at 0, 2, 4 and 6 hours. The taken samples (60 µl) were placed into 200 µl microtubes at -20 °C. Before their analysis, the samples were thermal ly degraded (60 min, 100 °C) to minimize the influence of various MW of the fragments cleaved away. Free reductive ends were determined by the reaction with the Somogyi and Nelson agent [9] with the following modification: 50 µl of the sample was mixed with the same volume of the Somogyi agent newly prepared. Following the stirring for 15 minutes the mixture was incubated in a thermoblock at 100 °C. After coo ling, 100 µl of the Nelson agent was added, the samples were stirred, centrifuged for a short period, 125 µl of the stained product was transferred into a microtitration 96-well plate and the absorbance at 540 nm was measured. After subtracting the background, the values of glucose equivalents (=number of reductive ends) were determined from a calibration curve.

**The results of degradations - the relation of rate of enzymatic reaction and the degree of the substitution of a derivative**

| | | | **concentration of free reductive ends (mM)** | | | |
|---|---|---|---|---|---|---|
| **derivative** | **degree of the substitution** | **precipitation bath** | **0 h** | **2 h** | **4 h** | **6 h** |
| Palmitoyl HA | 15 % | 20% water solution of lactic acid | 0.0026 | 0.8271 | 0.9501 | 1.2202 |
| Palmitoyl HA | 36 % | 20% water solution of lactic acid | 0 | 0.1826 | 0.3464 | 0.3826 |
| Palmitoyl HA | 52 % | 20% water solution of lactic acid | 0.0026 | 0.2014 | 0.2764 | 0.2943 |
| Stearoyl HA | 37 % | 20% water solution of lactic acid | 0.86 | 0.182 | 0.23 | 0.302 |
| Undecanoyl HA | 37 % | 20% water solution of lactic acid | 0.129 | 0.469 | 0.578 | 0.585 |

The conclusion is that the fibres prepared are biodegradable. The rate of the degradation depends on the derivative used and on its substitution degree.

### Example 29

Cytotoxicity of the derivatives and the fibres was studied with the use of the MTT test at mouse dermal fibroblasts culture 3T3 for 72 hours. Both the derivative and the fibres were weighed and immersed into isopropanol in a laminar box until isopropanol evaporated. Dry derivatives/fibres are transported to a growth medium (3600 µg/ml of fibres and 1000 µg/ml of a derivative) and kept to swell overnight. The fibres were disintegrated with the use of ultrathurax to obtain a homogenous suspension. The extract from the fibres or the filtrate of a mixture, filtered with the use of 100µm filter, were also tested but these modifications did not influence the viability, so the next fibres were tested only in the form of a disintegrated suspension.

The results of the tests of viability show that neither the derivative nor the prepared fibres influence the cell viability significantly.

### Example 30

The fibres were degraded with the use of hyaluronidase and esterase enzymes. 20 U of esterase and 200 U of hyaluronidase were added to 20 mg of fibre wicks in 750 µl of PBS. Fibres with the enzymes were kept at the temperature 37 °C for 72 hours. Then this mixture was used for affecting the cells. The concentrations tested were 1000, 500 and 100 µg/ml, according to the fibre concentration from which the supernatant was prepared. The cell line and viability test procedure are the same as in the Example 25. The procedure was performed 3 times independently and the data were statistically evaluated with the use of Student's t-test.

The results of the tests of viability show that the fibre degradation products also do not influence the cell viability significantly.

The results the tests of viability of products of degradation, where:

| **mark** | **a fibre according to the example no.** | **bath** |
|---|---|---|
| a | 3 | water solution of citric acid |
| b | 8 | water solution of tartaric acid |
| c | 12 | water solution of lactic acid |
| k | control | a fibre from native hyaluronane |

### Example 31

Tensile was tested mechanically with the use of one-column tensile strength tester Instron 3343; the used head of 100 N. During the test, first the fibre is pre-tightened at the speed of 1 mm.min⁻¹ up to the stress of 0.05 N. After the tightening, both the stress and deformation are reset automatically and then the test is performed at the speed of 10 mm.min⁻¹ until the fibre breaks. Tensile strength tests were performed at room temperature and room relative humidity.

The strength and deformation at break were calculated as a median of at least 5 valid measurements. The fibres reach the strength of 3 to 10 cN .tex⁻¹ depending on the concentration of derivative and the degree of the substitution and also on the drawing rate. The deformation reaches the values of 5 to 40 % depending on the above mentioned parameters. The tables show the values of strengths and tensibilities, these numbers are medial.

| | **prepared according to** | **Mw HA [kDa]** | **p Ka** | **strength [cN/dtex]** | **tensibility [%]** |
|---|---|---|---|---|---|
| Hydrophobized HA derivative | Example 1 | 253 | - | - | - |
| Formic acid | Example 27 | 88 | 3.75 | not possible to measure | |
| Acetic acid | Example 26 | 167 | 4.76 | 0.56 | 16.13 |
| Propionic acid | Example 25 | 193 | 4.88 | 0.30 | 11.00 |
| Ascorbic acid | Example 10 | 120 | 4.1 | 0.94 | 21.07 |
| Lactic acid | Example 12 | 137 | 3.86 | 0.58 | 21.90 |
| Malic acid | Example 11 | 130 | 3.46 | 0.75 | 25.83 |
| Citric acid | Example 5 | 106 | 3.15 | 0.78 | 20.53 |
| Tartaric acid | Example 8 | 95 | 3.01 | 0.49 | 16.49 |

The table with the values of strength and deformation of fibres prepared form hyaluronan in a similar way as in the Examples 12 and 13.

| **Bath** | **According to the example no.** | **Concentration [%]** | **Substitution degree [%]** | **Sheerness [tex]** | **Strength cN/dtex** | **Tensibility [%]** |
|---|---|---|---|---|---|---|
| 20% water solution of lactic acid | 13 | 5,7 | 52 | 12,2 | 0,93 | 39 |
| 20% water solution of lactic acid | 12 | 7 | 15 | 16,3 | 0,56 | 13 |
| 20% water solution of lactic acid | 12 | 6 | 41 | 15 | 0,55 | 37 |
| 20% water solution of lactic acid | 12 | 6 | 36 | 17 | 0,47 | 29 |
| 20% water solution of lactic acid | 13 | 6,3 | 48 | 11,2 | 0,86 | 20 |
| 20% water solution of lactic acid | 13 | 6,3 | 48 | 11 | 0,86 | 17 |

It results from the measurement of the mechanical properties of the fibres and from the experiments that the fibres can be processed by textile processing.

### Example 32

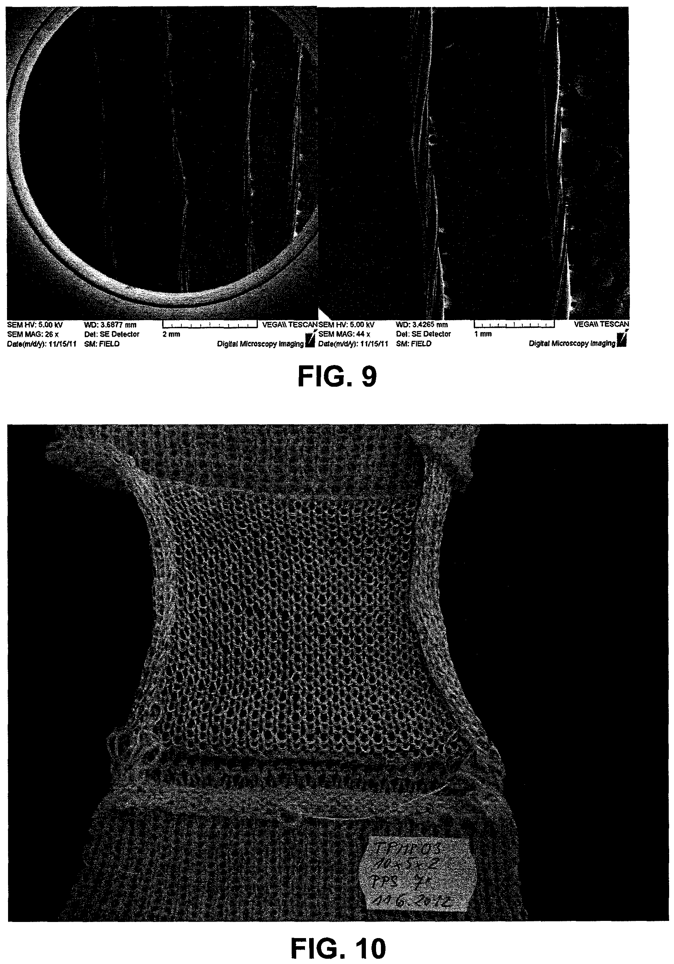

The fibres made of palmitoyl hyaluronan (fibres prepared in a similar way as in the Example 12) were twisted in a twisting apparatus with the spindle speed of 1400 RPM and the monofilament feeding speed of 4 m.min-1. The threads from palmitoyl hyaluronan are showed in Fig. 9.

### Example 33

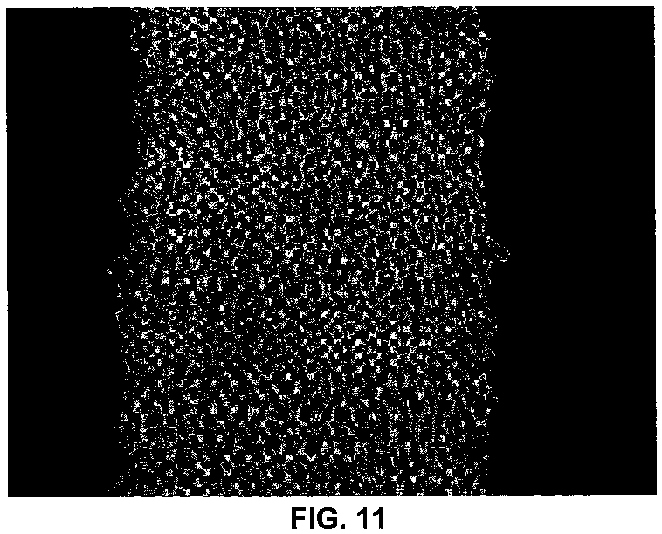

Fibres prepared in a similar way as in the Example 12 were textile processed in the flat operational weft-knitting machine Shima-Seiku with automatic needle selection into the form of one-face weft-knit fabric (Fig. 10) and the into the form of double weft-knit fabric (Fig. 11).

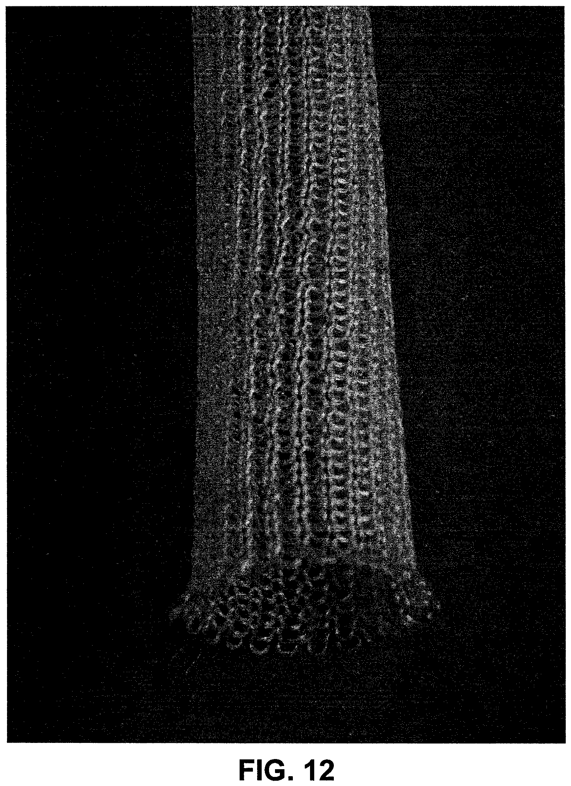

The fibres prepared in a similar way as in the Example 12 were textile processed in the tubular operational weft-knitting machine Harry Lucas into the form of tubular fabric (Fig. 12).

## Claims

1. A method of a preparation of fibres based on the hyaluronan derivative C₁₁ - C₁₈-acylated on hydroxyl groups of hyaluronic acid **characterized in that** it is prepared a spinning solution comprising an acylated derivative of hyaluronan of the concentration in the range of 0.01 % by weight to 15 % by weight in the mixture of water and 2-propanol, where water content is in a range of 30 % by volume to 90 % by volume, a content of 2-propanol is 10 % by volume to 70 % by volume, then the spinning solution is introduced to the coagulation bath comprising a water solution of an organic acid or its salts of alkali metals, or alkaline earth metals, wherein the acid is selected from the group comprising lactic, tartaric, citric, malic, ascorbic, oxalic acid or a combination thereof, to form fibres which are then washed with alcohol and dried.

2. The method of the preparation according to the claim 1 **characterized in that** the hyaluronan derivative is acylated preferably on the primary alcohol of N-acetyl-glucosamine.

3. The method of preparation according to the claim 1 or 2 **characterized in that** the hyaluronan derivative is selected from the group comprising the C₁₁ - C₁₂-acylated derivatives with the degree of the substitution of 30 % and more, C₁₃ - C₁₅- acylated derivatives with the degree of the substitution of 20 % and more, and C₁₆ - C₁₈- acylated derivatives with the degree of the substitution of 5 % and more.

4. The method of the preparation according to any of the preceding claims **characterized in that** the hyaluronan derivative is palmitoyl hyaluronan.

5. The method of the preparation according to any of the preceding claims **characterized in that** the concentration of the water solution of organic acid in the coagulation bath is 0.5 % to 30 % by weight for lactic acid, 0.5 % to 42 % by weight for citric acid, 0.5 % to 50 % by weight for malic acid, 0.5 % to 25 % by weight for ascorbic acid, 0.5 % to 60 % by weight for oxalic acid.

6. The method of the preparation according to any of the preceding claims **characterized in that** the coagulation bath is formed of the saturated water solution of organic acid.

7. The method of the preparation according to any of the preceding claims **characterized in that** the coagulation bath contains the saturated water solution of lactic acid.

8. The method of the preparation according to any of the preceding claims **characterized in that** the spinning solution is deaerated before its introducing into the coagulation bath.

9. The method of the preparation according to any of the preceding claims **characterized in that** the temperature of the coagulation bath is in the range of 15 to 45 °C and the time of keeping the fibres in the coagulation bath is in the range of 10 seconds to 24 hours.

10. The method of the preparation according to any of the preceding claims **characterized in that** the fibres are washed with alcohol selected from the group comprising ethanol, 1-propanol, and 2-propanol, and that optionally they are drawn after being washed with alcohol.

11. The fibre based on the hyaluronan derivative C₁₁ - C₁₈-acylated on hydroxyl groups of hyaluronic acid **characterized in that** its diameter is 50 to 300 µm and its tensile strength is in the range of 0.3 to cN/dtex and its elongation is in the range of 10 to 40 %.

12. The use of the fibre defined in the claim 11 for production of knitted, woven or nonwoven textiles.

13. The textile on the base of the fibres defined in the claim 11

14. The use of the textiles on the base of the fibres defined in the claim 11 for production of implantable surgical materials for human and veterinary medicine.

## Patentansprüche

1. Verfahren zur Herstellung von Fasern auf der Basis des auf den Hydroxylgruppen C₁₁ - C₁₈-acylierten Hyaluronans, **dadurch gekennzeichnet, dass** eine zu verspinnende Lösung vorbereitet wird, die das acylierte Hyaluronan mit der Konzentration im Bereich von 0,01 Gew.-% bis 15 Gew.-% in einem Gemisch von Wasser und Propan-2-ol enthält, wobei der Gehalt an Wasser 30 Vol.-% bis 90 Vol.-% und der Gehalt an Propan-2-ol 10 Vol.-% bis 70 Vol.-% beträgt, wonach die derart erhaltene zu verspinnende Lösung in ein Koagulierbad eingeleitet wird, das eine wässrige Lösung einer organischen Säure oder eines durch die Reaktion derartiger Säure mit alkalischen Metallen bzw. alkalischen Erdmetallen erhaltenen Salzes enthält, wobei die Säure aus der Gruppe ausgewählt ist, die Milchsäure, Weinsäure, Zitronensäure, Apfelsäure, Askorbsäure, Oxalsäure oder deren Kombinationen umfasst, wodurch Fasern entstehen, die anschließend mit einem Alkohol durchgewaschen und getrocknet werden.

2. Verfahren zur Herstellung nach Anspruch 1, **dadurch gekennzeichnet, dass** das Hyaluronan vorzugsweise auf dem primären Alkohol des N-Acetyl-Glukosamins acyliert ist.

3. Verfahren zur Herstellung nach Anspruch 1 oder 2, **dadurch gekennzeichnet, dass** das Hyaluronan aus der Gruppe ausgewählt ist, die C₁₁ - C₁₂-acylierte Hyaluronane mit einem Substitutionsgrad von mindestens 30 %, C₁₃ - C₁₅-acylierte Hyaluronane mit einem Substitutionsgrad von mindestens 20 % sowie C₁₆ - C₁₈-acylierte Hyaluronane mit einem Substitutionsgrad von mindestens 5 % umfasst.

4. Verfahren zur Herstellung nach einem der vorhergehenden Ansprüche, **dadurch gekennzeichnet, dass** das Hyaluronan Palmitoyl-Hyaluronan ist.

5. Verfahren zur Herstellung nach einem der vorhergehenden Ansprüche, **dadurch gekennzeichnet, dass** das die Konzentration der wässrigen Lösung der in dem Koagulierbad enthaltenen wässrigen Lösung einer organischen Säure 0,5 bis 30 Gew.-% für Milchsäure, 0,5 bis 42 Gew.-% für Zitronensäure, 0,5 bis 50 Gew.-% für Apfelsäure, 0,5 bis 25 Gew.-% für Askorbsäure, bzw. 0,5 bis 60 Gew.-% für Oxalsäure beträgt.

6. Verfahren zur Herstellung nach einem der vorhergehenden Ansprüche, **dadurch gekennzeichnet, dass** das Koagulierbad von einer gesättigten wässrigen Lösung einer organischen Säure gebildet ist.

7. Verfahren zur Herstellung nach einem der vorhergehenden Ansprüche, **dadurch gekennzeichnet, dass** das Koagulierbad eine gesättigte wässrige Lösung der Milchsäure enthält.

8. Verfahren zur Herstellung nach einem der vorhergehenden Ansprüche, **dadurch gekennzeichnet, dass** die zu verspinnende Lösung vor dem Einleiten in das Koagulierbad entgast wird.

9. Verfahren zur Herstellung nach einem der vorhergehenden Ansprüche, **dadurch gekennzeichnet, dass** die Temperatur des Koagulierbads in dem Bereich von 15 bis 45 °C liegt und die Verweilzeit der Fasern in dem Koagulierbad 10 Sekunden bis 24 Stunden beträgt.

10. Verfahren zur Herstellung nach einem der vorhergehenden Ansprüche, **dadurch gekennzeichnet, dass** die Fasern mit einem Alkohol durchgewaschen werden, der aus der Gruppe ausgewählt ist, die Ethanol, Propan-1-ol und Propan-2-ol umfasst, und dass die Fasern nach dem Waschen gegebenenfalls verstreckt werden.

11. Eine Faser auf der Basis des auf den Hydroxylgruppen C₁₁ - Cis-acylierten Hyaluronans, **dadurch gekennzeichnet, dass** sie den Durchmesser in dem Bereich von 50 bis 300 µm, die Zugfestigkeit in dem Bereich von 0,3 bis 1 cN/dtex und die Ziehfähigkeit in dem Bereich von 10 bis 40 % aufweist.

12. Verwendung der in dem Anspruch 11 definierten Faser zur Herstellung von gestrickten, gewebten und ungewebten Textilien.

13. Eine Textilie auf der Basis der in dem Anspruch 11 definierten Fasern.

14. Verwendung der Textilien auf der Basis der in dem Anspruch 11 definierten Fasern zur Herstellung von implantierbaren chirurgischen Materialien auf den Gebieten der humanen und veterinären Medizin.

## Revendications

1. Procédé de préparation des fibres à base d'hyaluronane C₁₁ - C₁₈-acylé sur les groupes hydroxyles de l'hyaluronane, **caractérisé en ce qu'**on prépare une solution de filage comprenant de l'hyaluronane acylé de concentration comprise entre 0,01 % en poids et 15 % en poids dans un mélange d'eau et de 2-propanol, où la teneur en eau étant comprise entre 30 % vol. et 90 % vol., la teneur en 2-propanol étant comprise entre 10 % vol. et 70 % vol., puis la solution de filage est introduite dans un bain de coagulation comprenant une solution aqueuse d'acide organique ou de son sel alcalin ou alcalino-terreux, l'acide étant choisi dans le groupe comprenant l'acide lactique, tartrique, citrique, malique, ascorbique, oxalique ou leur combinaison, en formant des fibres qui sont ensuite lavées avec de l'alcool et séchées.

2. Procédé de préparation selon la revendication 1, **caractérisé en ce que** l'hyaluronane est acylé de préférence sur l'alcool primaire de N-acétyl-glucosamine.

3. Procédé de préparation selon la revendication 1 ou 2, **caractérisé en ce que** l'hyaluronane est choisi dans le groupe comprenant les hyaluronanes C₁₁-C₁₂-acylés avec un degré de substitution 30 % et supérieur, les hyaluronanes C₁₃ - C₁₅-acylés avec le degré de substitution 20 % et supérieur et les hyaluronanes C16 - C18- acylés avec le degré de substitution 5 % et supérieur.

4. Procédé de préparation selon l'une quelconque des revendications précédentes, **caractérisé en ce que** l'hyaluronane est le palmitoyl hyaluronane.

5. Procédé de préparation selon l'une quelconque des revendications précédentes, **caractérisé en ce que** la concentration de la solution aqueuse de l'acide organique dans le bain de coagulation est comprise pour l'acide lactique entre 0,5 % et 30 % en poids, pour l'acide citrique entre 0,5 % et 42 % en poids, pour l'acide malique entre 0,5 % et 50 % en poids, pour l'acide ascorbique entre 0,5 % et 25 % en poids, pour l'acide oxalique entre 0,5 % et 60 % en poids.

6. Procédé de préparation selon l'une quelconque des revendications précédentes, **caractérisé en ce que** le bain de coagulation est constitué d'une solution aqueuse saturée d'acide organique.

7. Procédé de préparation selon l'une quelconque des revendications précédentes, **caractérisé en ce que** le bain de coagulation est constitué d'une solution aqueuse saturée d'acide lactique.

8. Procédé de préparation selon l'une quelconque des revendications précédentes, **caractérisé en ce que** la solution de filage est dégazée avant d'être introduite dans le bain de coagulation.

9. Procédé de préparation selon l'une quelconque des revendications précédentes, **caractérisé en ce que** la température du bain de coagulation est comprise entre 15 et 45°C et le temps de séjour des fibres dans le bain de coagulation est compris entre 10 secondes et 24 heures.

10. Procédé de préparation selon l'une quelconque des revendications précédentes, **caractérisé en ce que** les fibres sont lavées avec de l'alcool choisi dans le groupe comprenant l'éthanol, le 1-propanol et le 2-propanol, et qu'elles sont, après le lavage avec de l'alcool, éventuellement étirées.

11. Fibre à base d'hyaluronane C₁₁ - C₁₈-acylé sur les groupes hydroxyles de l'hyaluronane, **caractérisée en ce que** son diamètre est compris entre 50 et 300 µm et sa résistance à la traction est comprise entre 0,3 et 1 cN/dtex et son allongement est compris entre 10 et 40 %.

12. Utilisation de la fibre définie dans la revendication 11 pour la fabrication des textiles tricotés, tissés ou non tissés.

13. Textile à base des fibres définies dans la revendication 11.

14. Utilisation des textiles à base des fibres définies dans la revendication 11 pour la fabrication des matériaux chirurgicaux implantables pour la médecine humaine et vétérinaire.
